# EUROPEAN PATENT SPECIFICATION

(11) **EP 2 403 495 B1**
(45) Date of publication and mention of the grant of the patent: **20.05.2015**
(21) Application number: 10712519.7
(22) Date of filing: 04.03.2010
(51) Int. Cl.: A61K 31/4425, A61K 31/4015, A61K 31/4415, A61P 1/16

(54) **METADOXINE FOR USE AS INHIBITOR OF HEPATIC FIBROSIS.**
METADOXIN ZUR VERWENDUNG ALS MITTEL ZUR UNTERDRÜCKUNG VON LEBERFIBROSE
MÉTADOXINE DESTINÉE À ÊTRE UTILISÉE EN TANT QU'INHIBITEUR D'UNE FIBROSE HÉPATIQUE

(30) Priority: 06.03.2009 IT MI20090333
(43) Date of publication of application: 11.01.2012
(73) Proprietor: Laboratori Baldacci SPA, 56100 Pisa (IT)
(72) Inventor: BALDACCI, Massimo, I-56125 Pisa (IT)
(74) Representative: Bartorelli, Luisa
(86) International application number: PCT/IB2010/050930
(87) International publication number: WO 2010/100619

(56) References cited:
- MURIEL PABLO ET AL: "Fibrosis and glycogen stores depletion induced by prolonged biliary obstruction in the rat are ameliorated by metadoxine." LIVER INTERNATIONAL, vol. 23, no. 4, August 2003 (2003-08), pages 262-268, XP002552816 ISSN: 1478-3223
- ANNONI G ET AL: "PYRIDOXOL L 2-PYRROLIDON-5 CARBOXYLATE PREVENTS ACTIVE FIBROPLASIA IN CARBON TETRACHLORIDE TREATED RATS" PHARMACOLOGICAL RESEARCH, vol. 25, no. 1, 1992, pages 87-94, XP008114043 ISSN: 1043-6618
- AROSIO B ET AL: "Changes in expression of the albumin, fibronectin and type I procollagen genes in CCl-4-induced liver fibrosis: Effect of pyridoxol L,2-pyrrolidon-5 carboxylate" PHARMACOLOGY AND TOXICOLOGY, vol. 73, no. 6, 1993, pages 301-304, XP002552817 ISSN: 0901-9928
- MURIEL P ET AL: "Metadoxine partially prevents fibrosis and completely preserves glycogen stores in prolonged biliary obstruction in the rat: A comparison with colchicine." JOURNAL OF HEPATOLOGY, vol. 38, no. Supplement 2, April 2003 (2003-04), page 79, XP002552818 & 38TH ANNUAL MEETING OF THE EUROPEAN ASSOCIATION FOR THE STUDY OF THE LIVER; INSTANBUL, TURKEY; MARCH 29-APRIL 01, 2003 ISSN: 0168-8278
- MEDINA J ET AL: "Approach to the pathogenesis and treatment of nonalcoholic steatohepatitis" DIABETES CARE 200408 US, vol. 27, no. 8, August 2004 (2004-08), pages 2057-2066, XP002584426 ISSN: 0149-5992
- CACCIATORE L ET AL: "METADOXINE TREATMENT OF FATTY LIVER ASSOCIATED WITH CHRONIC HEPATITIS" CLINICAL TRIALS JOURNAL, vol. 25, no. 3, 1988, pages 220-226, XP008114048 ISSN: 0009-9325
- INNERARITY T L ET AL: "Disparities in the interaction of rat and human lipoproteins with cultured rat fibroblasts and smooth muscle cells. Requirements for homology for receptor binding activity", JOURNAL OF BIOLOGICAL CHEMISTRY 1980 US, vol. 255, no. 23, 1980, pages 11163-11172, ISSN: 0021-9258
- SUKEZANE T ET AL: "Human diploid fibroblasts are resistant to MEK/ERK-mediated disruption of the actin cytoskeleton and invasiveness stimulated by Ras", ONCOGENE 20050825 GB, vol. 24, no. 36, 25 August 2005 (2005-08-25), pages 5648-5655, ISSN: 0950-9232
- Y. Liu ET AL: "Animal models of chronic liver diseases", American journal of physiology: Gastrointestinal and liver physiology, vol. 304, no. 5, 28 December 2012 (2012-12-28), pages G449-G468, XP055162740, ISSN: 0193-1857, DOI: 10.1152/ajpgi.00199.2012

## Description

The present invention is defined in the appended claims.

Subject-matters which are not encompassed by the scope of the claims do not form part of the present invention.

The object of the present invention is represented by the therapeutic use of metadoxine in the treatment of hepatic fibrosis.

### Background art

Metadoxine, or Pyridoxol L-2-pyrrolidone-5-carboxylate, whose structure formula is reported hereinbelow 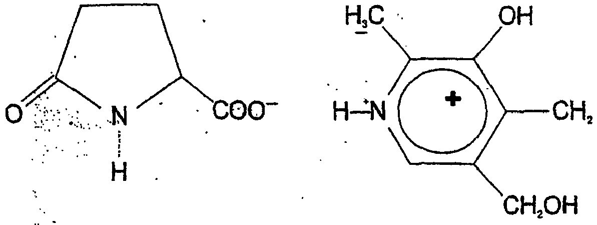 is known for its effectiveness in acute and chronic alcoholism and for the prevention of alcohol related pathology (1, 2, 3); metadoxine is currently sold as active ingredient of the Metadoxil drug. The ability of metadoxine to accelerate the elimination of alcohol from plasma and from tissues (4), to reduce the damages alcohol-induced on the structure and functionality level of the hepatocyte is well documented, together with its effectiveness against neuropsychological disorders present in chronic alcoholics (5, 6).

Nevertheless, up to date it has never been shown that such substance can intervene on the biomechanical mechanisms that give origin to fibrosis, which represents the key pathogenetic outcome of all the hepatic diseases evolving towards the cirrhosis of any etiology (7).

The most recent data reported in the literature recognizes fibrogenesis as the common mechanism of hepatic pathology developing due to inflammation, associated with the presence of hepatic viruses, immunological processes or predisposing factors such as diabetes, obesity, hyperlipidemia and an incorrect diet (8, 9).

Hepatic fibrosis is an excessive accumulation of extracellular protein matrix in the presence of any one chronic inflammatory disease.

The progression of hepatic fibrosis is very slow and the plasmatic parameters that are normally carried out for evaluating hepatic functionality are not universally accepted as indicators of fibrosis severity and vary slightly with the progression of the disease. Various non-invasive methods have been proposed that are useful for predicting the degree of hepatic fibrosis, but only the hepatic biopsy with the histological exam is considered the "gold standard" parameter that identifies the severity and the progression of the fibrosis.

The study of the genesis and evolution of hepatic fibrosis from a clinical standpoint is difficult to approach and consequently the treatment is usually aimed to eliminate the causes of chronic inflammation due to viral infection, autoimmune phenomena or metabolic alterations. Removing the causes of the inflammatory process (e.g. elimination of the virus) can slow the fibrogenesis or even bring the structural modifications that originated in the liver back to normality. However, in some cases, the fibrosis can develop into cirrhosis and lead to severe clinical complications which require liver transplant even when the causes of chronic inflammation are eliminated.

There is no standard treatment for hepatic fibrosis. The ideal antifibrotic treatment consists of a substance which should be liver-specific, well tolerated even when administered over long time periods and effective in reducing the excessive deposition of collagen without leading to modifications of the normal metabolism of the hepatic stellate cells.

Among the drugs which are proposed as antifibrotic agents, there are the corticosteroids, due to their anti-inflammatory activity, the antioxidants (vitamin E, S-adenosylmethionine), the phosphodiesterase inhibitors (pentoxyphylline) and the inhibitors of the renin-angiotensin system.

It is universally known that fibroblasts are the greatest producers of collagen in the liver that is subjected to chronic inflammatory episodes. The model of human fibroblasts *in vitro* therefore represents the most standardized method for showing the proliferation of these cells and for the analysis of the mediators involved in cellular development.

For this reason, we have conducted an *in vitro* study, which is detailed in the Experimental Part of this document, in order to show the inhibitory effect of metadoxine on hepatic fibrogenesis.

The experimental results obtained and described in the Experimental Part of this document show the antifibrotic activity of metadoxine, which could therefore be used in all the hepatic pathology forms in which the hepatic damage is due to a fibrotic process with the evolution of the disease towards more severe pathologies such as cirrhosis or hepatic carcinoma.

### Experimental section

The methods used allow evaluating the effect of substances provided with potential antifibrotic activity on the proliferation of fibroblasts.

A line of immortalized human fibroblasts was used, maintained in a culture in a suitable medium.

In order to carry out the proliferation experiments, the fibroblasts were detached from the flask by means of trypsinization, counted and distributed on 96-well flat-bottom plates at the concentration of 2000 cells/100µl medium.

Scalar concentrations of metadoxine were added (range of concentration from 10⁻¹¹M to 10⁻³M).

The plates were maintained in CO₂ incubator at 37°C. The proliferation of the cells was measured at 24 h intervals up to 120h by using the Mosmann method (10), based on the spectrophotometer determination of the conversion of MTT [3-(4,5 dimethylthiazole-2-yl)-2-5 diphenyltetrazolium bromide] to its blue reduction product, formazan, by mitochondrial enzymes of the metabolically active cells. The optical densities (O.D.) were determined by means of the Titertek Multiscan (Flow) spectrophotometer with 540nm filter.

The O.D.s of the samples are expressed as percentages of the samples with respect to the control group. Metadoxine was added in various concentrations (10⁻¹¹M - 10⁻³M range) and at 10⁻⁵M concentration the effect of the studied product on the growth of fibroblasts as metabolically active cells, capable of affecting the synthesis of fibrinogen, is clear.

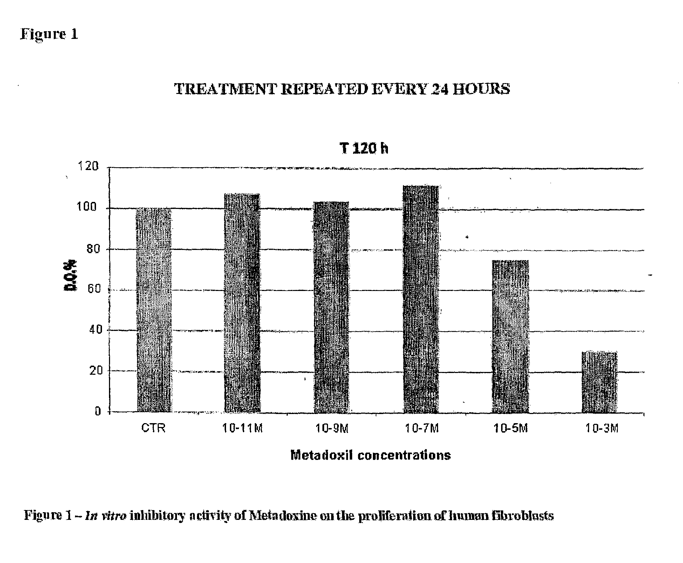

In figure 1, the anti-fibrotic activity of metadoxine is reported, expressed in percent with respect to the control group, measured after 120h from the first treatment, with treatments repeated every 24h, according to the standardized technique (9).

As can be shown from the obtained experimental results, metadoxine is capable of inhibiting the fibrogenic activity of fibroblasts already at 10⁻⁵M concentrations. Such experimental model is normally used for testing the anti-fibrotic activity of substances which also result effective in human hepatic pathology.

Our data demonstrates that metadoxine possesses an anti-fibrotic activity in a range of concentrations comprised between 10⁻⁶M and 10⁻⁵M. Such activity is comparable to that of corticosteroids and is perhaps even greater. Metadoxine can therefore be employed for such activity as an inhibitor of hepatic fibrogenesis.

### Conclusions

The results obtained with this *in vitro* study (the percentage values reported in the figure represent the average of three experimental tests) are of undoubted scientific interest due to the fact that they show the inhibitory activity of metadoxine on hepatic fibrogenesis. It should be underlined that the concentrations at which the product is effective are 2-3 lower than those that are obtained *in vivo* in the patient orally treated with 500 mg metadoxine dose, in conventional pathologies.

In addition, the optimal tolerability of the metadoxine product has been well known on the market for many years, and it has been used as a protector drug against alcohol-induced tissue damages; its present use as a molecule that can affect hepatic fibrogenesis acquires further therapeutic significance.

The object of the present invention is therefore represented by metadoxine in the treatment of hepatic fibrosis, preferably non-alcohol dependent (NASH) and, in particular, in pharmaceutical formulations based on metadoxine usable for the treatment of hepatic fibrosis.

Metadoxine results effective in hepatic fibrosis due to an acute and/or chronic inflammatory state of the liver; such pathology can also be associated with the presence of hepatic viruses, with hyperlipidemia, with hyperglycemia and/or with other altered metabolic factors.

Further object of the present invention is therefore represented by metadoxine in the treatment of hepatic fibrosis associated with an acute and/or chronic inflammatory state of the liver and/or with the presence of hepatic viruses, with hyperlipidemia, with hyperglycemia and/or with other altered metabolic factors. In the treatment of hepatic fibrosis, metadoxine and/or the formulations containing it are orally administered, preferably to a human.

A further aspect of the invention can be specified in the formulations containing metadoxine which are preferably in tablet, capsule and oral solution form, and have a metadoxine content comprised between 50 and 1000 mg per dose, preferably between 300 and 600 mg per dose.

Furthermore, in the treatment of hepatic fibrosis, the aforesaid dosage comprised between 50 and 1000 mg of metadoxine per dose, preferably between 300 and 600 mg per dose, is preferably administered by means of a dosage 2 or 3 times per day.

### Bibliography

1) Addolorato G, Ancona C, Capisto E, Gasbarrini G: Metadoxine in the treatment of acute and chronic alcoholism: a review.
2) Diaz Martinez MCLR, Diaz Martinez A, Villamil Salcedo V, Cruz Fuentes C: Efficacy in the management of acute alcohol intoxication. J Int Med Res, 2002; 30: 44-51.
3) Santoni S, Corradini P, Zocchi M, Camarri F: La metadoxina nella patologia alcol-correlata. La Clinica Terapeutica, 1989; 130: 115-122.
4) Calabrese V, Carlino S, Chinnici V, De Bernardis E, Rizza V: La metadoxina modula le cinetiche di assorbimento, metabolismo ed eliminazione dell'etanolo. Alcologia, 1986; 5(1-2).
5) Intaschi G, Lattanzi L, Lombardi G, Panzera V, Guazzelli M, Maggini C: Gli effetti della metadoxina nella dipendenza da alcol: implicazioni per lo studio dei rapporti tra alcolismo e depressione. Progressi in Psichiatria - Estratti dal Congresso - Roma 6-11 febbraio 1989.
6) Carboni MA, Corsi R: Uso terapeutico della metadoxina nei disturbi psichici e comportamentali alcol-correlati. Clin Ter, 1987; 123: 469-473.
7) Wallace K, Burt AD, Wright MC: Liver fibrosis. Biochem J, 2008; 411:1-18.
8) Pinzoni M, Rombouts K: Liver Fibrosis: from the bench to clinical targets. Digestive and Liver Disease, 2004; 36: 231-242.
9) Bataller, R, Bremer DA: Liver fibrosis. J Clin Inv, 2005; 115: 209-218.

## Claims

1. Metadoxine for use in the treatment of hepatic fibrosis, **characterized in that** said metadoxine is to be orally administered to a human.

2. Metadoxine for use according to claim 1, **characterized in that** said hepatic fibrosis is non-alcohol dependent fibrosis (NASH).

3. Metadoxine for use according to claim 1, **characterized in that** said hepatic fibrosis is generated by an acute or chronic inflammatory state of the liver.

4. Metadoxine for use according to claim 1, **characterized in that** said hepatic fibrosis is associated with the presence of hepatic viruses, hyperlipidemia, hyperglycemia and/or other altered metabolic factors.

5. Metadoxine for use according to claim 1, **characterized in that** said formulation has a metadoxine content comprised between 50 and 1000 mg per dose.

6. Metadoxine for use according to claim 5, **characterized in that** said formulation has a metadoxine content comprised between 300 and 600 mg per dose.

7. Metadoxine for use according to claims 1 to 6, **characterized in that** said dose is administered 2 or 3 times per day.

8. Metadoxine for use according to claims 1 to 7, **characterized in that** said formulation is in tablet, capsule or solution form.

## Patentansprüche

1. Metadoxin zur Verwendung bei der Behandlung von Leberfibrose, **dadurch gekennzeichnet, dass** das Metadoxin einem Menschen oral verabreicht werden soll.

2. Metadoxin zur Verwendung nach Anspruch 1, **dadurch gekennzeichnet, dass** die Leberfibrose eine alkoholunabhängige Fibrose (NASH) ist.

3. Metadoxin zur Verwendung nach Anspruch 1, **dadurch gekennzeichnet, dass** die Leberfibrose durch einen akuten oder chronischen Entzündungszustand der Leber ausgelöst ist.

4. Metadoxin zur Verwendung nach Anspruch 1, **dadurch gekennzeichnet, dass** die Leberfibrose mit dem Auftreten von hepatischen Viren, Hyperlipidämie, Hyperglykämie und/oder anderen veränderten metabolischen Faktoren verknüpft ist.

5. Metadoxin zur Verwendung nach Anspruch 1, **dadurch gekennzeichnet, dass** die Formulierung einen Metadoxingehalt hat, der zwischen 50 und 1000 mg pro Dosis enthalten ist.

6. Metadoxin zur Verwendung nach Anspruch 5, **dadurch gekennzeichnet, dass** die Formulierung einen Metadoxingehalt hat, der zwischen 300 und 600 mg pro Dosis enthalten ist.

7. Metadoxin zur Verwendung nach den Ansprüchen 1 bis 6, **dadurch gekennzeichnet, dass** die Dosis 2 bis 3 Mal täglich verabreicht wird.

8. Metadoxin zur Verwendung nach den Ansprüchen 1 bis 7, **dadurch gekennzeichnet, dass** die Formulierung in Tabletten-, Kapsel- oder Lösungsform ist.

## Revendications

1. Métadoxine pour une utilisation dans le traitement d'une fibrose hépatique, **caractérisée en ce que** ladite métadoxine doit être administrée par voie orale à un être humain.

2. Métadoxine pour une utilisation selon la revendication 1, **caractérisée en ce que** ladite fibrose hépatique est une fibrose non alcoolique (NASH).

3. Métadoxine pour une utilisation selon la revendication 1, **caractérisée en ce que** ladite fibrose hépatique est générée par un état inflammatoire aigu ou chronique du foie.

4. Métadoxine pour une utilisation selon la revendication 1, **caractérisée en ce que** ladite fibrose hépatique est associée à la présence de virus hépatiques, d'hyperlipidémie, d'hyperglycémie et/ou d'autres facteurs métaboliques altérés.

5. Métadoxine pour une utilisation selon la revendication 1, **caractérisée en ce que** ladite formulation a une teneur en métadoxine comprise entre 50 et 1000 mg par dose.

6. Métadoxine pour une utilisation selon la revendication 5, **caractérisée en ce que** ladite formulation a une teneur en métadoxine comprise entre 300 et 600 mg par dose.

7. Métadoxine pour une utilisation selon les revendications 1 à 6, **caractérisée en ce que** ladite dose est administrée 2 ou 3 fois par jour.

8. Métadoxine pour une utilisation selon les revendications 1 à 7, **caractérisée en ce que** ladite formulation est sous forme de comprimés, de capsules ou de solution.
